# EUROPEAN PATENT SPECIFICATION

(11) **EP 3 344 255 B1**
(45) Date of publication and mention of the grant of the patent: **28.08.2019**
(21) Application number: 17771894.7
(22) Date of filing: 30.05.2017
(51) Int. Cl.: A61K 31/5415, A01N 43/84, A61P 31/04

(54) **ANTIMICROBIAL EFFECT OF METHYLENE BLUE ON COLISTIN RESISTANT ACINETOBACTER BAUMANNII BACTERIA**
ANTIMIKROBIELLE WIRKUNG VON METHYLENBLAU AUF COLISTIN-RESISTENTEN ACINETOBACTER BAUMANNII-BAKTERIEN
EFFET ANTIMICROBIEN DU BLEU DE MÉTHYLÈNE SUR LES BACTÉRIES ACINETOBACTER BAUMANNII RÉSISTANTES À LA COLISTINE

(30) Priority: 01.06.2016 TR 201607319
(43) Date of publication of application: 11.07.2018
(73) Proprietor: Gazel, Deniz, Gaziantep (TR); Otkun, Muserref, Canakkale (TR); Akcali, Alper, Canakkale (TR)
(72) Inventor: Gazel, Deniz, Gaziantep (TR); Otkun, Muserref, Canakkale (TR); Akcali, Alper, Canakkale (TR)
(74) Representative: Handanoglu, Erdal
(86) International application number: PCT/TR2017/050227
(87) International publication number: WO 2017/209719

(56) References cited:
- WO-A1-2010/130007
- RU-C1- 2 363 470
- J. H. MOFFATT ET AL: "Colistin Resistance in Acinetobacter baumannii Is Mediated by Complete Loss of Lipopolysaccharide Production", ANTIMICROBIAL AGENTS AND CHEMOTHERAPY, vol. 54, no. 12, 20 September 2010 (2010-09-20), pages 4971-4977, XP055150102, ISSN: 0066-4804, DOI: 10.1128/AAC.00834-10 cited in the application

## Description

### Field of Invention

The invention is related to the use of methylene blue for inhibition of colistin-resistant Acinetobacter baumannii bacteria.

### Description of Known Art

Acinetobacter baumannii is a gram negative, immobile, oxidase negative and non-fermenter microorganism. Acinetobacter baumannii treatment is becoming more difficult due to its increasing multiple drug resistance. Commonly found in hospital environments, especially in intensive care units, as it remains viable for days in external environments it can cause severe epidemics. These multiple resistant, non-fermenting gram negative bacteria commonly cause infections like pneumonia, injury, skin infection, urinary system infections, bacteremia and meningitis in severely ill patients in hospitals. A.baumannii is frequently on the agenda due to recent multiple drug resistance and globally has become a bacteria that is difficult to treat.

A.baumannii may form biofilms which makes the bacterium resistant to antibiotics. Additionally this agent may gain antibiotic resistance with beta-lactamases, efflux pump systems, enzymatic inactivation of drugs and ribosomal ribonucleic acid mutations.

A.baumannii, as with other gram negative bacteria, has an outer membrane surrounding the cytoplasmic membrane. The outer membrane has a lipopolysaccharide structure, formed of hydrophobic lipid A, core layer with polysaccharide structure and hydrophilic O antigens. The external membrane of A.baumannii contains a variety of proteins, similar to those seen in other gram negative bacteria. These proteins in the outer membrane ensure selectivity of the cell wall, while loss of these proteins plays an important role in the development of antimicrobial resistance.

Colistin (polymyxin E) is an old antibiotic that has gained importance again for treatment of multiple drug resistant microorganisms. Colistin is a peptide structure antibiotic used in the treatment of A.baumannii. However, bacteria show resistance to colistin. Clinical studies in Turkey have reported up to 27.5% colistin resistance rates in Acinetobacter baumannii isolates while other researchers have reported up to 40% in Spain and 28% in Korea. This resistance is proposed to occur due to modifications of the polysaccharides in the outer membrane causing reduced affinity in the target region for the antibiotic. This resistance to colistin has brought the use of antibiotic combinations to inhibit A.baumannii to the agenda. Colistin has been used in double or triple combinations with antibiotics like rifampin, tigecycline and vancomycin.

As mentioned for colistin, antibiotic mixtures prepared in combination with other antibiotic varieties are available for gram negative and gram positive bacteria. The forms and locations of use for colistin are listed in patent number GB1402947, giving different antibiotic varieties like colistin, tylosin and erythromycin for use against gram negative and gram positive bacteria, and patent number US2010160215 related to antibiotic compositions created to treat gram negative bacterial infections. WO2010130007 discloses polymyxin analogs for the treatment of bacterial infections caused by polymyxin (e. g. colistin) resistant A. baumannii.

RU2363470 discloses the antimicrobial activity of compositions comprising an antibiotic and methylene blue against multidrug-resistant staphylococci, enterobacteria, corynebacteria, Klebsiella and Candida.

Moffatt et al., 2010, discloses that the resistance of A. baumannii against colistin is linked to the loss of LPS in the outer membrane of these gram negative bacteria.

However, there is a need to develop new antimicrobials that are more effective, with fewer side effects, low cost and easy administration and especially that will affect colistin resistant or colistin susceptible (but carbapenem resistant) A.baumannii bacteria.

The invention that is the topic of this application aims to complete this required development with methylene blue. Methylene blue is a chemical with molecular formula C₁₆H₁₈N₃SCl. This chemical is normally used in microbiological science for bacterial staining, in internal medicine as an antidote to carbon monoxide poisoning, as drug for oral candidiasis and in surgical sciences for staining of the surgical field. For carbon monoxide poisoning, 10 mg/ml intravenous flacon forms and 65-130 mg tablet forms are available for use. Additionally acollutory dropper bottle form with 15 ml solution containing 150 mg methylene blue is used as mouthwash. For other uses, available forms include intravenous and tablet forms for systemic infections and dropper bottle forms for local infections.

### Detailed Explanation of Invention

Starting from the current description of the art, the aim of the invention is to use methylene blue to kill Acinetobacter baumannii with colistin resistance. The aim is to use methylene blue as an antibiotic against Acinetobacter baumannii strains that have become resistant to colistin. Under normal conditions Acinetobacter baumannii bacteria are resistant to methylene blue. However, the intense use of colistin against A.baumannii bacteria under clinical conditions has led to the occurrence of colistin resistant mutant bacteria.

Colistin resistance occurring in A.baumannii organisms is thought to form as a result of mutations in two gene regions with a role in synthesis of the lipopolysaccharide layer of the cell membrane (lipid A) (Moffat et al., Antimicrobial Agents and Chemotherapy, 54:4971-77, Aralik 2010)(Adams et al., Antimicrobial Agents and Chemotherapy, 53: 3628-34, Eylül 2009). The first mechanism is loss of the lipopolysaccharide layer with disruption of lipopolysaccharide biosynthesis due to mutations of the lpxA, lpxC and lpxD genes. The second mechanism is related to ethanolamine modification of the lipid A component and increased positive charge on the lipopolysaccharide layer as a result of mutations at the PmrAB locus of the PmrA/B two-component system. The result of both mechanisms is that the lipid structure of the cell membrane that colistin normally affects becomes ineffective. In short, A.baumannii becomes colistin resistant after transformation of the cell wall. Developing resistance against colistin due to disruption of the lipopolysaccharide structure of the cell membrane, these bacteria may be called mutant bacteria. It is understood from investigations that this mutant bacteria, which normally is resistant to methylene blue, has this resistance lost during the formation of resistance to colistin. In other words, while this lipopolysaccharide layer is lost or thinned, the bacteria lose their selectively permeable structure and become susceptible to methylene blue, which they are resistant to under normal conditions.

The conduct and results of these investigations are explained below.

Firstly, colistin-susceptible A.baumannii strains obtained from the same passage were subcultured on media (Mueller Hinton Agar) containing 14 mcg/ml methylene blue and without methylene blue and colistin gradient test (E-test) strips were placed on them. Growth was observed in these two media. It was identified that methylene blue did not inhibit growth of colistin susceptible strains. However, it appeared that in the medium containing methylene blue the susceptibility of A.baumannii strain to colistin was slightly increased. The results of this study showed that the MIC values for colistin susceptible A.baumannii strains in medium containing methylene blue regressed by a low level like 0.5 mcg/ml. For slightly increased susceptibility of colistin susceptible Acinetobacter baumannii bacteria, the MIC (minimum inhibitory concentration) value should be higher than 0.5 mcg/ml (at least 1 mcg/ml). Our studies showed that in A.baumannii strains with colistin MIC values of 0.5 mcg/ml or less there was no increased susceptibility to colistin with the effect of methylene blue.

Later, the same tests were performed on A.baumannii bacteria with resistance to colistin. While there was growth in the medium without methylene blue (Mueller Hinton Agar), there was no growth in the medium containing methylene blue. From this result it is understood that methylene blue inhibits colistin resistant A.baumannii bacteria. These bacterial varieties with resistance to colistin were tested based on species with different levels of MIC (minimum inhibitory concentrations) for colistin. Firstly, the results of observations of bacteria with high levels of colistin resistance (MIC value 32 mcg/ml) found that methylene blue completely stopped growth of A.baumannii bacteria. In other words, it's understood these bacteria were inhibited. The results of observations with bacteria with low levels of colistin resistance (MIC value 1 or 3 mcg/ml) observed that methylene blue did not completely stop growing of A.baumannii bacteria but ensured a clear reduction in the resistance to colistin (to 0.5 mcg/ml level). The probable cause of this reduction is that those with MIC values more than 0.5 mcg/ml in the bacterial population were selectively killed by methylene blue. Additionally this methylene blue solution was observed to display inhibitory effects on all bacterial subpopulations with MIC value above 0.5 mcg/ml and not just those bacteria with MIC value 32 mcg/ml. However, the inhibition rate for strains with high MIC value (like 32 mcg/ml) was higher than the inhibition rate for strains with low MIC value (like 4-8 mcg/ml). In other words, the susceptiblity to methylene blue is proportional to the colistin resistance levels of colistin-resistant A.baumannii bacteria.

In light of the information above and as a result of studies, for complete destruction or reduction of resistance of colistin-resistant A.baumannii bacteria, a common MIC limit value may be determined. The results of our studies show that for complete loss of resistance or lowering of resistance levels by methylene blue, the MIC values for these colistin resistant bacteria should be higher than 2 mcg/ml, in other words 4 mcg/ml is sufficient.

These effects of inhibition of colistin-resistant A.baumannii bacteria by methylene blue are clearly understood from the appearance on MHA (Mueller Hinton Agar) media. Image 1 and 2 provide the appearance of A.baumannii strains with homogeneous resistance to colistin on MHA (Mueller Hinton Agar) media. The strain with 32 mcg/ml MIC value shown in Image 1 is observed to be completely inhibited with 14 mcg/ml methylene blue as shown in Image 2. Images 3 and 4 provide images of heteroresistant A.baumannii strains with resistance to colistin on MHA. On Image 3, the MHA appearance of double-zoned heteroresistant strain is given, while in 14 mcg/ml methylene blue the internal zone with MIC of 32 mcg/ml is totally inhibited and the outer zone with MIC of 2 mcg/ml becomes susceptible with MIC value observed to regress to 0.5 mcg/ml.

This inactivation caused by methylene blue is thought to occur because methylene blue easily infiltrates the cell interior of the resistant bacterial population with disrupted lipopolysaccharide layer, inhibiting the vital functions of the bacteria.

Methylene blue is preferably dissolved in water. The methylene blue solution used is chosen as 14 mcg/ml concentration; however in clinical drug use this rate may change depending on the bacterial resistance levels and patient's situation.

The use of methylene blue for inhibition of colistin-resistant A.baumannii bacteria may be appropriate for intravenous, intrathecal, oral or topical use depending on the different infection regions.

Additionally, methylene blue solution may be used for disinfection of surgical tools, probes, catheters, intensive care apparatus and in hospital environments against A.baumannii bacteria. Killing colistin-resistant strains will reduce intensive care epidemics, and will ensure increase in the susceptibility to colistin of colistin-susceptible or heteroresistant strains.

In short, this invention is related to the use of methylene blue to inhibit of Acinetobacter baumannii organisms with high level colistin resistance.

The problem of lack of inhibition of A.baumannii bacteria with resistance to colistin will be solved by the use of methylene blue. For methylene blue inhibition of A.baumannii with high levels or low levels of colistin resistance, the colistin MIC (minimum inhibitory concentration) values of these bacteria should be at least 4 mcg/ml. For methylene blue to increase the susceptibility of colistin-resistant A.baumannii bacteria, the MIC values should be at least 1 mcg/ml. Apart from these uses of methylene blue, the use of methylene blue for disinfection of surgical tools, probes, catheters, intensive care apparatus and hospital environments against colistin resistant or colistin susceptible Acinetobacter baumannii organisms is also relevant.

In summary, the invention is defined by the appended claims and it comprises:
Methylene blue for use in therapeutic killing of Acinetobacter baumannii organisms with colistin resistance.

Methylene blue for use in therapeutic killing of Acinetobacter baumannii organisms with colistin resistance, wherein the colistin MIC (minimum inhibitory concentration) of Acinetobacter baumannii bacteria resistant to colistin is at least 4 mcg/ml.

Methylene blue for use in therapeutic killing of Acinetobacter baumannii organisms with colistin resistance, wherein the used methylene blue is a solution with water.

Methylene blue for use in therapeutic killing of Acinetobacter baumannii organisms with colistin resistance, wherein concentration of used methylene blue is 14 mcg/ml.

Methylene blue for use in therapeutic killing of Acinetobacter baumannii organisms with colistin resistance, in the form of intravenous, intrathecal, oral or topical.

Use of methylene blue for disinfection of surgical tools, probes, catheters, intensive care apparatus and hospital environments (patient beds, surface of patient rooms) against colistin resistant Acinetobacter baumannii organisms.

The use of methylene blue for disinfection of surgical tools, probes, catheters, intensive care apparatus and hospital environments (patient beds, surface of patient rooms) against colistin resistant Acinetobacter baumannii organisms, wherein the used methylene blue is a solution with water.

The use of methylene blue for disinfection of surgical tools, probes, catheters, intensive care apparatus and hospital environments (patient beds, surface of patient rooms) against colistin resistant Acinetobacter baumannii organisms, wherein concentration of used methylene blue is 14 mcg/ml.

### Description of Images:

**Image-1** Appearance of A.baumannii strain with homogeneous resistance to colistin on MHA (Mueller Hinton Agar)
**Image-2** Appearance of A.baumannii strain with homogeneous resistance to colistin on MHA (Mueller Hinton Agar) with methylene blue added
**Image-3** Appearance of A.baumannii strain with heterogenous resistance to colistin on MHA (Mueller Hinton Agar)
**Image-4** Appearance of A.baumannii strain with heterogenous resistance to colistin on MHA (Mueller Hinton Agar) with methylene blue added

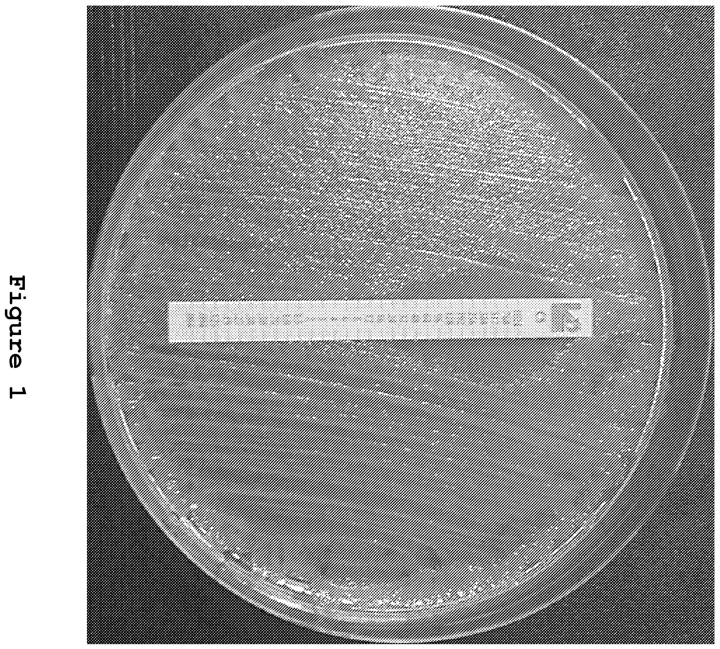

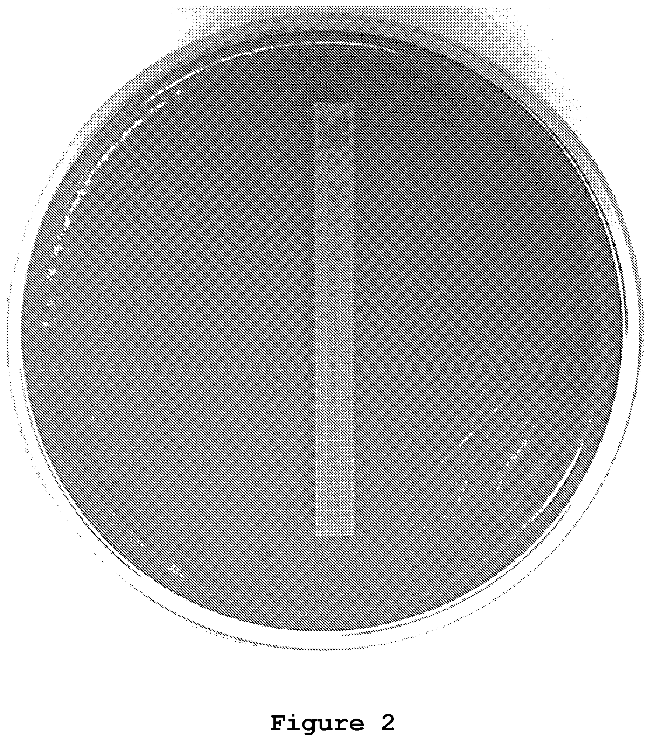

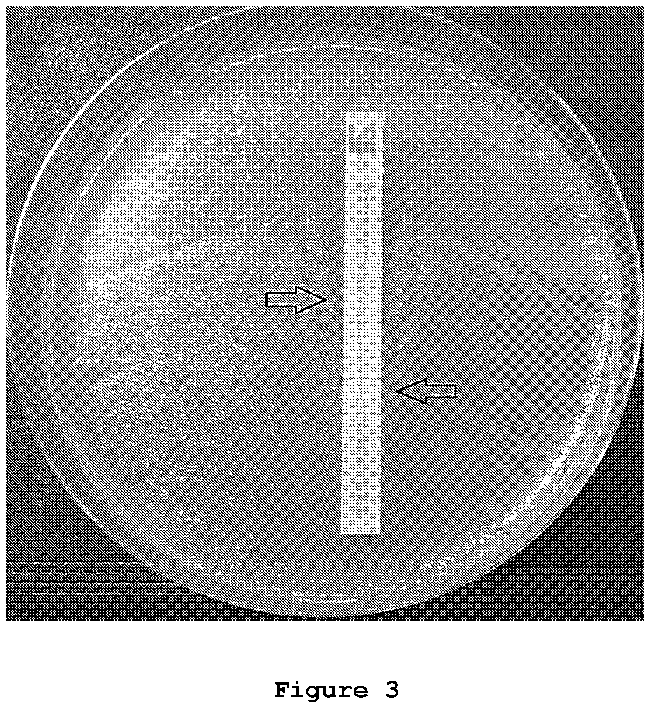

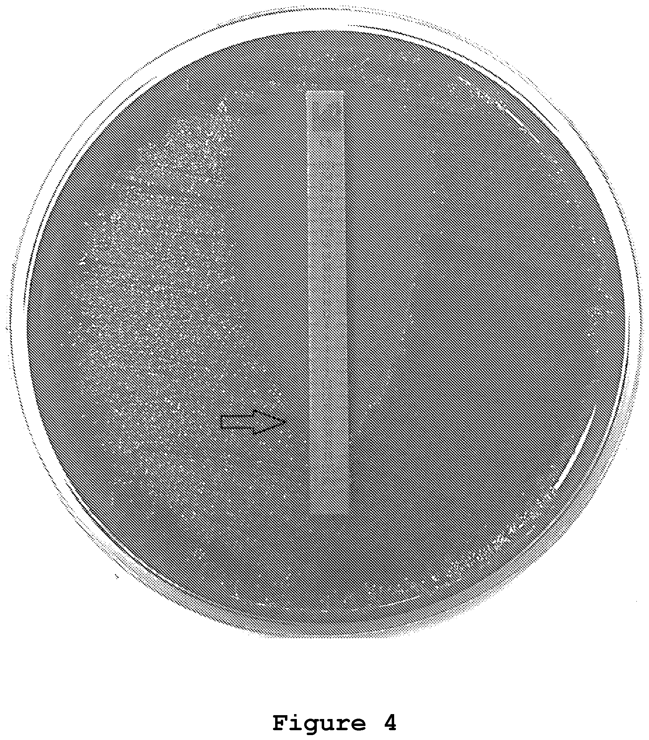

## Claims

1. Methylene blue for use in therapeutic killing of Acinetobacter baumannii organisms with colistin resistance.

2. Methylene blue for use according to claim 1, wherein the colistin MIC (minimum inhibitory concentration) of Acinetobacter baumannii bacteria resistant to colistin is at least 4 mcg/ml.

3. Methylene blue for use according to claim 1, wherein the used methylene blue is a solution with water.

4. Methylene blue for use according to claim 1, wherein concentration of used methylene blue is 14 mcg/ml.

5. Methylene blue for use according to claim 1, in the form of intravenous, intrathecal, oral or topical.

6. Use of methylene blue for disinfection of surgical tools, probes, catheters, intensive care apparatus and hospital environments (patient beds, surface of patient rooms) against colistin resistant Acinetobacter baumannii organisms.

7. The use of methylene blue according to claim 6, wherein the used methylene blue is a solution with water.

8. The use of methylene blue according to claim 6, wherein concentration of used methylene blue is 14 mcg/ml.

## Patentansprüche

1. Methylenblau zur therapeutischen Abtötung von Acinetobacter baumannii-Organismen mit Colistin-Resistenz.

2. Methylenblau zur Verwendung nach Anspruch 1, wobei die Colistin-MHK (minimale Hemmkonzentration) der gegen Colistin resistenten Acinetobacter baumannii-Bakterien mindestens 4 µg / ml beträgt.

3. Methylenblau zur Verwendung nach Anspruch 1, wobei das verwendete Methylenblau eine Lösung mit Wasser ist.

4. Methylenblau zur Verwendung nach Anspruch 1, wobei die Konzentration an verwendetem Methylenblau 14 µg / ml beträgt.

5. Methylenblau zur Verwendung nach Anspruch 1, in Form von intravenös, intrathekal, oral oder topisch.

6. Verwendung von Methylenblau zur Desinfektion von chirurgischen Instrumenten, Sonden, Kathetern, Intensivpflegegeräten und Krankenhausumgebungen (Patientenbetten, Oberfläche von Patientenzimmern) gegen Colistin-resistente Acinetobacter baumannii-Organismen.

7. Verwendung von Methylenblau nach Anspruch 6, wobei das verwendete Methylenblau eine Lösung mit Wasser ist.

8. Verwendung von Methylenblau nach Anspruch 6, wobei die Konzentration von verwendetem Methylenblau 14 µg / ml beträgt.

## Revendications

1. Bleu de méthylène pour être utilisé dans la destruction thérapeutique des organismes Acinetobacter baumannii avec résistance à la colistine.

2. Bleu de méthylène pour utilisation selon la revendication 1, dans lequel la CMI de la colistine (concentration minimale inhibitrice) des bactéries Acinetobacter baumannii qui résistent à la colistine est d'au moins 4 pg/ml.

3. Bleu de méthylène pour utilisation selon la revendication 1, dans lequel le bleu de méthylène utilisé est une solution avec de l'eau.

4. Bleu de méthylène pour utilisation selon la revendication 1, dans lequel la concentration de bleu de méthylène utilisé est de 14 pg/ml.

5. Bleu de méthylène pour utilisation selon la revendication 1 sous la forme intraveineuse, intrathécale, orale ou topique.

6. Utilisation de bleu de méthylène pour la désinfection des outils chirurgicaux, des sondes, des cathéters, des appareils de soins intensifs et des environnements hospitaliers (lits de patients, surface des chambres des patients) contre les organismes résistants à la colistine Acinetobacter baumannii.

7. Utilisation du bleu de méthylène selon la revendication 6, dans laquelle le bleu de méthylène qui est utilisé est une solution avec de l'eau.

8. Utilisation du bleu de méthylène selon la revendication 6, dans laquelle la concentration du bleu de méthylène qui est utilisé est de 14 pg/ml.
